# EUROPEAN PATENT SPECIFICATION

(11) **EP 4 054 769 B1**
(45) Date of publication and mention of the grant of the patent: **15.11.2023**
(21) Application number: 20789550.9
(22) Date of filing: 06.10.2020
(51) Int. Cl.: B05B 1/14, B05B 15/40

(54) **SPRAY NOZZLE CHIP**
SPRITZDÜSENCHIP
PUCE DE BUSE DE PULVÉRISATION

(30) Priority: 06.11.2019 EP 19207343
(43) Date of publication of application: 14.09.2022
(73) Proprietor: SHL Medical AG, 6302 Zug (CH)
(72) Inventor: SÄLL, Daniel, 131 28 Nacka Strand (SE); RENSTAD, Rasmus, 131 28 Nacka Strand (SE)
(86) International application number: PCT/EP2020/077990
(87) International publication number: WO 2021/089263

(56) References cited:
- WO-A1-2018/219798
- CA-A1- 3 057 403
- CN-U- 206 828 092
- US-A1- 2012 012 105

## Description

### TECHNICAL FIELD

The present invention relates to spray nozzle chips, for example for medicament delivery devices.

### BACKGROUND

A nozzle device may be configured to atomise a liquid, i.e. to make an aerosol of the liquid. A nozzle device of this type may comprise a substrate having a sieve or filter side provided with a filter for filtering out any undesired larger particles contained in the liquid to be atomised. The substrate may also have a spray-side provided with a spray-membrane having a plurality of orifices. The spray-membrane and the filter are configured to be in liquid communication. In the process of atomisation, the liquid first passes the filter where a slight pressure drop is obtained. The filtered liquid subsequently passes through the orifices of the membrane, whereby the liquid is atomised. An example of such a nozzle device is disclosed in WO2O18/219798 A1. The nozzle device comprises: a substrate, a sieve-side membrane comprising a plurality of sieve-side orifices, the sieve-side membrane being provided on a sieve-side of the substrate, a spray-side membrane comprising a plurality of spray-side orifices, the spray-side membrane being provided on a spray-side of the substrate, wherein the substrate has a first cavity portion extending to the sieve-side membrane, and a second cavity portion extending from the first cavity portion to the spray-side membrane, thereby providing fluid communication, along a fluid communication axis, between the sieve-side orifices and the spray-side orifices, the first cavity portion having a larger cross-sectional area than a cross-sectional area of the second cavity portion, the cross-sections being with respect to the fluid communication axis.

### SUMMARY

For some diseases such as respiratory diseases, e.g. asthma, it has been found that increased adherence to prescribed treatments and improved administration techniques will help control symptoms and reduce the risk of complications.

An object of the present invention is to provide a spray nozzle chip which solves or at least mitigates problems of the prior art.

There is hence according to a first aspect of the present invention provided a spray nozzle chip comprising: a substrate having a spray side and a sieve side, a spray membrane provided on the spray side, a sieve membrane provided on the sieve side, wherein the spray membrane is provided with spray orifices and the sieve membrane is provided with sieve orifices, wherein the substrate has a fluid channel which connects the spray orifices with the sieve orifices, and a pressure sensing device configured to measure deformation of the spray membrane to obtain a measure of pressure on the spray membrane.

It can thereby be detected whether an aerosol dispensing has been performed. In the event that the spray nozzle chip is mounted in an inhaler, the performance of the user may be monitored.

According to one embodiment the spray orifices are provided in a spray orifice region of the spray membrane, and wherein the pressure sensing device is configured to detect deformation of the spray orifice region. Pressure changes in the spray orifice region may thereby be detected. According to one embodiment the pressure sensing device comprises one of a strain gauge sensor, a capacitive pressure sensor and a piezoresistive pressure sensor.

According to one embodiment the pressure sensing device is a microelectromechanical system (MEMS) pressure sensor.

According to one embodiment the pressure sensing device is arranged on the spray membrane.

One embodiment comprises a reference pressure sensing device, wherein the substrate has a closed cavity which is delimited by a reference pressure region of the spray membrane, wherein the reference pressure sensing device is configured to measure deformation of the reference pressure region. In case the reference pressure sensing device is installed in an inhaler, as the user inhales, the reference pressure region will be is deflected towards the user's mouth. The reference pressure sensing device may thereby facilitate monitoring of the inhalation technique of a user.

According to one embodiment the cavity is a vacuum cavity.

According to one embodiment the reference pressure sensing device comprises one of a strain gauge sensor, a capacitive pressure sensor and a piezoresistive pressure sensor.

According to one embodiment the reference pressure sensing device is a MEMS pressure sensor.

According to one embodiment the reference pressure sensing device is arranged on the spray membrane.

There is according to a second aspect of the present invention provided a spray nozzle device comprising: the spray nozzle chip according to the first aspect, and a contact interface configured to be electrically connected to the pressure sensing device to supply power to and obtain pressure measurement signals from the pressure sensing device.

One embodiment comprises the spray nozzle chip including the reference pressure sensing device, wherein the contact interface is configured to be electrically connected to the reference pressure sensing device to supply power to and obtain pressure measurement signals from the reference pressure sensing device.

There is according to a third aspect of the present invention provided an aerosol dispenser comprising the spray nozzle device according to the second aspect.

According to one embodiment the aerosol dispenser is a medicament delivery device.

According to one embodiment the medicament delivery device is an inhaler or eye dispenser.

Generally, all terms used in the claims are to be interpreted according to their ordinary meaning in the technical field, unless explicitly defined otherwise herein. All references to "a/an/the element, apparatus, component, means, etc. are to be interpreted openly as referring to at least one instance of the element, apparatus, component, means, etc.", unless explicitly stated otherwise.

### BRIEF DESCRIPTION OF THE DRAWINGS

The specific embodiments of the present invention will now be described, by way of example, with reference to the accompanying drawings, in which:
Fig. 1 is a perspective view of an example of a spray nozzle chip depicting a spray side thereof;
Fig. 2 is a perspective view of the spray nozzle chip in Fig. 1 showing a sieve side thereof;
Fig. 3 shows a longitudinal cross section of the spray nozzle chip;
Fig. 4 is a perspective view of the spray nozzle chip mounted to a contact interface;
Fig. 5 is a perspective view of the spray nozzle chip mounted to the contact interface from another perspective;
Fig. 6 depicts the spray nozzle chip and contact interface mounted to a carrier;
Fig. 7 shows the carrier in Fig. 6 from another perspective;
Fig. 8 shows a perspective view of a spray nozzle device;
Fig. 9 is a cross sectional view of the spray nozzle device in Fig. 8;
Fig. 10 is a top view of the spray nozzle device in Fig. 8; and
Fig. 11 shows a side view of an aerosol dispenser comprising the spray nozzle device in Fig. 8.

### DETAILED DESCRIPTION

The embodiments of the present invention as defined by the appended claims will now be described more fully hereinafter with reference to the accompanying drawings, in which exemplifying embodiments are shown. The present invention may, however, be embodied in many different forms and should not be construed as limited to the embodiments set forth herein, as long as the resulting embodiments are covered by the present invention as defined by the appended claims; rather, these embodiments are provided by way of example so that this disclosure of the present invention will be thorough and complete, and will fully convey the scope of the present invention as defined by the appended claims to those skilled in the art.

Like numbers refer to like elements throughout the description.

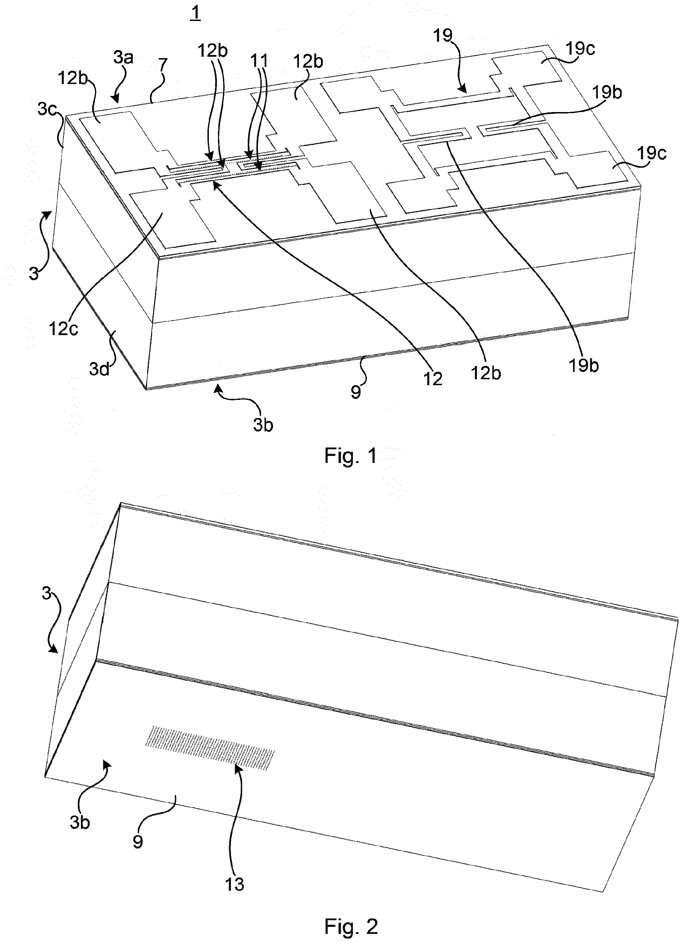

Fig. 1 shows an example of a spray nozzle chip 1. The spray nozzle 1 may be configured to be used in an aerosol dispenser. The spray nozzle 1 may be configured to be arranged in an aerosol dispenser.

The spray nozzle chip 1 comprises a substrate 3. The substrate 3 has a spray side 3a and a sieve side 3b. The spray side 3a and the sieve side 3b are arranged opposite to each other.

The substrate 3 may for example comprise a ceramic material such as silicon. The exemplified substrate 3 comprises a spray side substrate 3c and a sieve side substrate 3d which are joined or bonded, and which form the substrate 3. Alternatively, the substrate 3 could be made of a single piece of substrate material.

The spray nozzle chip 1 comprises a spray membrane 7. The spray membrane 7 may for example comprise silicon or a nitride. The spray membrane 7 is provided on the spray side 3a of the substrate 3. The spray membrane 7 may be bonded with the substrate 3; or formed by etching through the substrate 3 directly. The spray membrane 7 comprises a plurality of spray orifices 11. The spray membrane 7 has a spray orifice region comprising the spray orifices 11.

The spray nozzle chip 1 comprises a sieve membrane 9. The sieve membrane 9 may for example comprise silicon or a nitride. The sieve membrane 9 is provided on the sieve side 3b of the substrate 3. The sieve membrane 9 is bonded with the substrate 3, or formed by etching through the substrate 3 directly. The sieve membrane 9 comprises a plurality of sieve orifices 13, shown in Fig. 2.

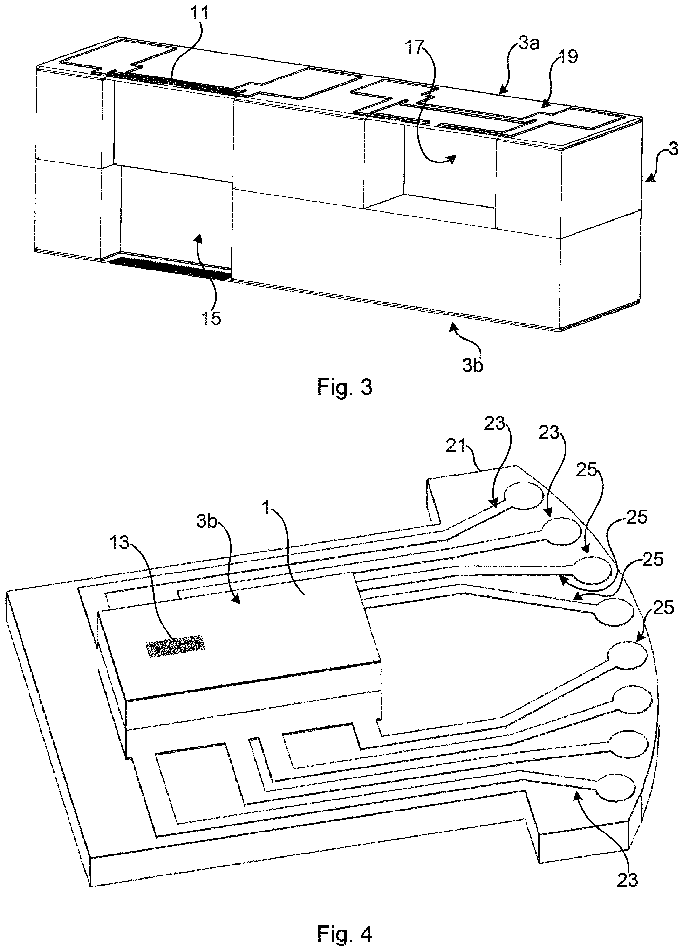

Fig. 3 shows a section of the spray nozzle chip 1. The substrate 3 has a fluid channel 15, which extends through the substrate 3 from the spray side 3a to the sieve side 3b. The fluid channel 15 connects the spray orifices 11 with the sieve orifices 13. The fluid channel 15 is configured to set the spray orifices 11 in fluid communication with the sieve orifices 13.

The spray orifice region is defined by the borders or boundary formed by the inner walls of the fluid channel 15 relative to the spray membrane 7.

Turning back to Fig. 1, the spray nozzle chip 1 comprises a pressure sensing device 12. The pressure sensing device 12 is configured to detect deformation of the spray membrane 7. The amount of deformation of the spray membrane 7 provides a measure of the pressure that the spray membrane 7 is subjected to. The pressure sensing device 12 is arranged on the spray membrane 7.

The pressure sensing device 12 is configured to detect deformation of the spray orifice region of the spray membrane 7. The pressure sensing device 12 or a portion thereof is arranged on the spray orifice region of the spray membrane 7.

The pressure sensing device 12 may be a MEMS pressure sensor.

In the example shown in Fig. 1, the pressure sensing device 12 is a strain gauge. The pressure sensing device 12 comprises a Wheatstone bridge. The pressure sensing device 12 comprises resistive elements 12b which form the Wheatstone bridge. The resistive elements 12b are provided on the spray orifice region of the spray membrane 7. The resistive elements 12b may for example extend adjacent to and parallel with the spray orifices 11. The pressure sensing device 12 may comprise contact pads 12c in electrical contact with the resistive elements 12b. The contact pads 12c may be configured to be electrically connected to a power source and configured to relay current flow i.e. pressure measurement signals from the resistive elements 12b. Any deformation of the resistive elements 12b causes a change in electrical resistance of the resistive elements 12b. This in turn causes a change in the magnitude of the current flow through the Wheatstone bridge. The change in the current flow provides a measure of the deformation and hence of a pressure applied to the spray membrane 7.

The pressure sensing device 12 may alternatively to a strain gauge comprise a capacitive pressure sensor or a piezoresistive pressure sensor.

In the example of the spray nozzle chip 1 shown in Fig. 3, the substrate 3 comprises a closed cavity or chamber 17. The cavity 17 is delimited by the spray membrane 7. The cavity 17 is delimited by a reference pressure region of the spray membrane 7. The spray membrane 7 hence forms the wall on one side of the cavity 17. The other walls defining the cavity 17 may be formed by the substrate 3. The cavity 17 may be a vacuum cavity. The cavity 17 may hence contain a vacuum. The cavity 17 could alternatively be a pressurised chamber, i.e. a chamber which is not vacuum.

The exemplified spray nozzle chip 1 comprises a reference pressure sensing device 19, also depicted in Fig. 1. The reference pressure sensing device 19 is provided on the spray membrane 7. The reference pressure sensing device 19 is provided on the reference pressure region of the spray membrane 7. The reference pressure sensing device 19 is configured to measure deformation of the reference pressure region of the spray membrane 7.

According to the example shown in Figs 1 and 3, the reference pressure sensing device 19 is a strain gauge. The reference pressure sensing device 19 comprises a Wheatstone bridge. The reference pressure sensing device 19 comprises reference resistive elements 19b which form the Wheatstone bridge. The reference resistive elements 19b are provided on the reference pressure region of the spray membrane 7. The reference pressure sensing device 19 may comprise reference contact pads 19c in electrical contact with the reference resistive elements 19b. The reference contact pads 19c may be configured to be electrically connected to a power source and configured to relay current flow i.e. pressure measurement signals from the reference resistive elements 19b. Any deformation of the reference resistive elements 19b causes a change in electrical resistance of the reference resistive elements 19b. This in turn causes a change in the magnitude of the current flow through the Wheatstone bridge. The change in the current flow provides a measure of the deformation of the spray membrane 7 in the reference pressure region. The reference pressure sensing device 19 can be used for monitoring inhalation when the spray nozzle chip 1 is mounted in an inhaler. When the user inhales over the spray nozzle chip 1 the reference pressure region will be subjected to a suction force, deflecting or causing a deformation of the reference pressure region. The reference pressure sensing device 19 will thereby provide a measure of the user's inhalation technique.

The reference pressure sensing device 19 may alternatively to a strain gauge comprise a capacitive pressure sensor or piezoresistive pressure sensor.

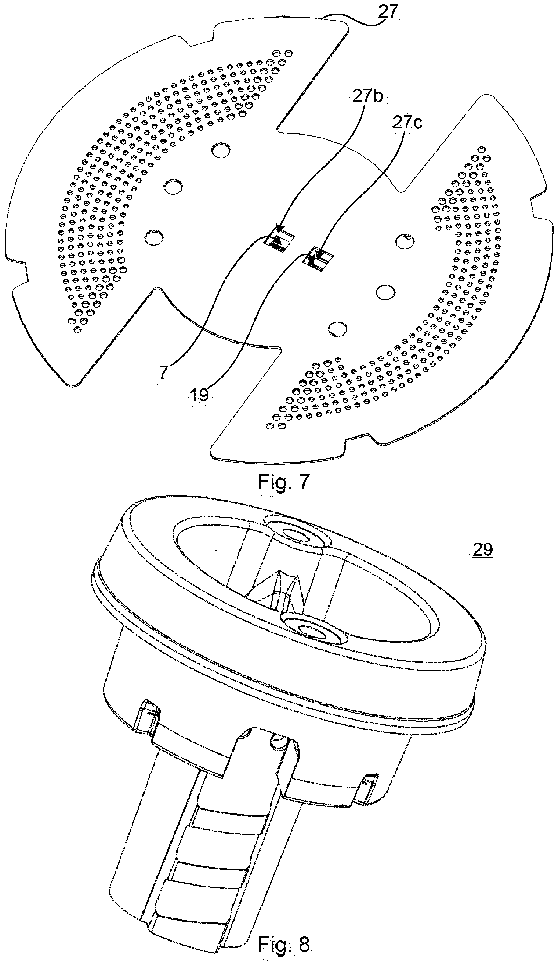

Fig. 4 shows the spray nozzle chip 1 mounted to a contact interface 21. The spray nozzle chip 1 and the contact interface 21 form part of a spray nozzle device 29 shown in Fig. 8. The contact interface 21 is a substrate. The contact interface 21 may for example comprise ceramic. The contact interface 21 comprises first conductive paths 23 configured to electrically connected to the pressure sensing device 12. The first conductive paths 23 may be connected to the contact pads 12b. The first conductive paths 23 may be configured to carry current to the pressure sensing device 12 and to transport current or pressure measurement signals from the pressure sensing device 12.

The contact pads 12b may for example be connected to the first conductive paths 23 by soldering or conductive glue.

The contact interface 21 comprises second conductive paths 25 configured to be electrically connected to the reference pressure sensing device 19. The second conductive paths 25 may be connected to the reference contact pads 19b. The second conductive paths 25 may be configured to carry current to the reference pressure sensing device 19 and to transport current or pressure measurement signals from the reference pressure sensing device 19.

The reference contact pads 19b may for example be connected to the second conductive paths 25 by soldering or conductive glue.

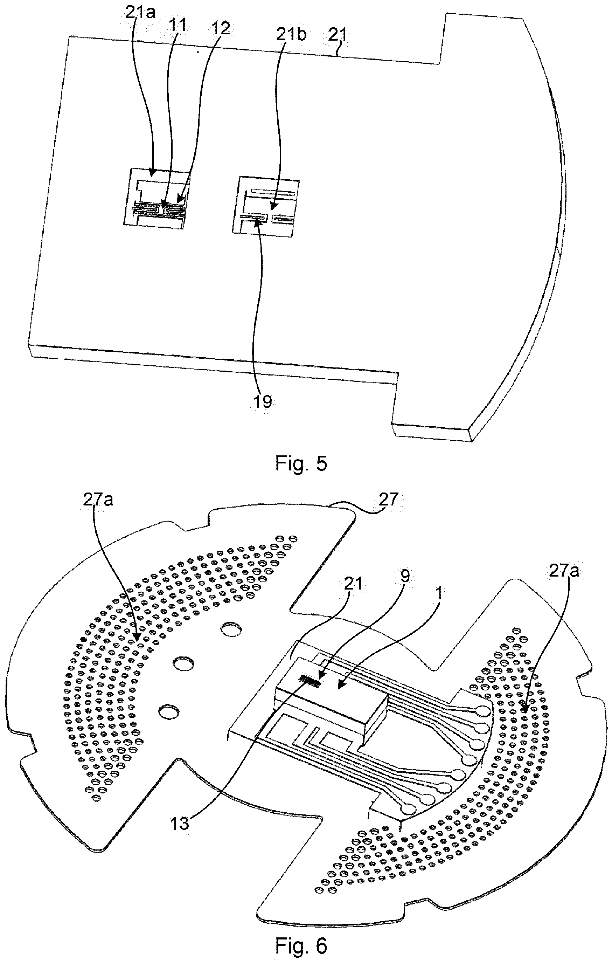

Fig. 5 depicts the opposite side of the contact interface 21 relative to the side shown in Fig. 4. In the present example, the spray membrane 7 faces the contact interface 21. The contact interface 21 is provided with a first through-opening 21a. The first through-opening 21 is aligned with the spray orifice region of the spray membrane 7. The spray orifices 11 are arranged within the first through-opening 21. The spray orifices 11 are accessible via the first through-opening 21 of the contact interface 21.

The contact interface 21 is provided with a second through-opening 21b. The second through-opening 21b is aligned with the reference pressure region of the spray membrane 7. The reference pressure region is hence accessible via the second through-opening 21b. Part of the reference pressure sensing device 19 is arranged within the second through-opening 21b.

Fig. 6 shows a bottom side of a contact interface carrier 27. The contact interface 21 is mounted onto the contact interface carrier 27. The contact interface 21 and the contact interface carrier 27 may alternatively be integrated as one part. The contact interface carrier 21 forms part of the spray nozzle device 29. The contact interface carrier 27 comprises a plurality of through-openings 27a distributed around the contact interface 21. The through-openings 27a may provide protective airflow for the spray nozzle device.

Fig. 7 shows a top side of the contact interface carrier 27. The contact interface carrier 27 has a carrier first through-opening 27b. The carrier first through-opening 27b is centred on the contact interface carrier 27. A central axis of the contact interface carrier 27 hence extends through the carrier first through-opening 27b. The carrier first through-opening 27b is aligned with the spray orifice region of the spray membrane 7 and the first through-opening 21a. The carrier first through-opening 27b hence leads to the spray orifice region of the spray membrane 7. The spray orifices 11 are hence accessible through the carrier first through-opening 27b and the first through-opening 21a.

The contact interface carrier 27 has a carrier second through-opening 27c. The carrier second through-opening 27c is aligned with the reference pressure region of the spray membrane 7 and the second through-opening 21b. The carrier second through-opening 27c hence leads to the reference pressure region. The reference pressure sensing device 19 is hence accessible via the carrier second through-opening 27c.

Fluid flow through the spray orifices 11 is hence enabled. Moreover, the reference pressure sensing device 19 is able to detect deformation of the reference pressure region due to deformation of the reference pressure region caused by suction force generated by user inhalation.

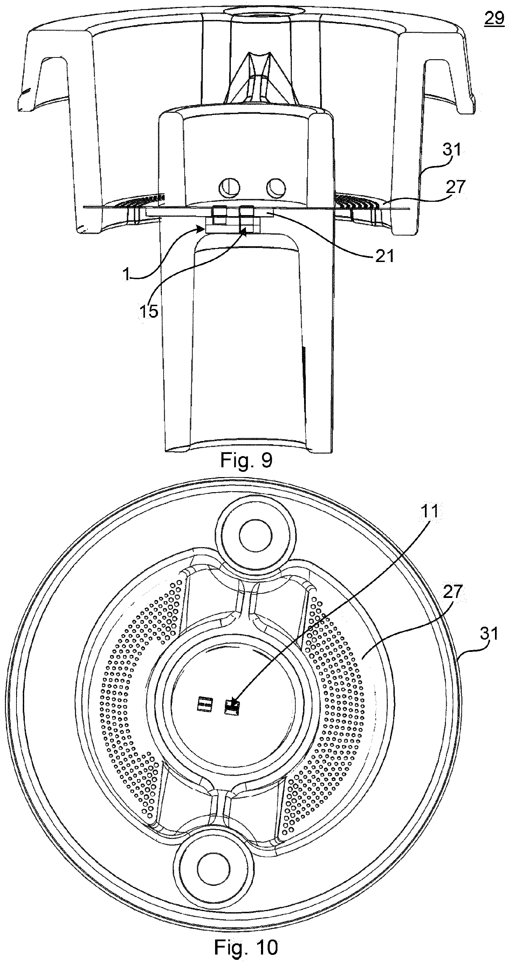

Fig. 8 shows a perspective view of a spray nozzle device 29. The spray nozzle device 29 is configured to be installed in an aerosol dispenser. The spray nozzle device 29 comprises a holding member 31 and the spray nozzle chip 1. Fig. 9 is a cross-section of the spray nozzle device 29. The holding member 31 is configured to hold the contact interface carrier 27. The contact interface carrier 27 is centred such that the central axis of the contact interface carrier 27 coincides with the central longitudinal axis of the spray nozzle device 29. The fluid channel 15 is hence centred in the spray nozzle device 29. The central longitudinal axis of the spray nozzle device 29 thus extends through the fluid channel 15, as shown in Fig. 10.

The spray nozzle chip 1 and/or the contact interface carrier 27 may be moulded into the main body of the spray nozzle device 29.

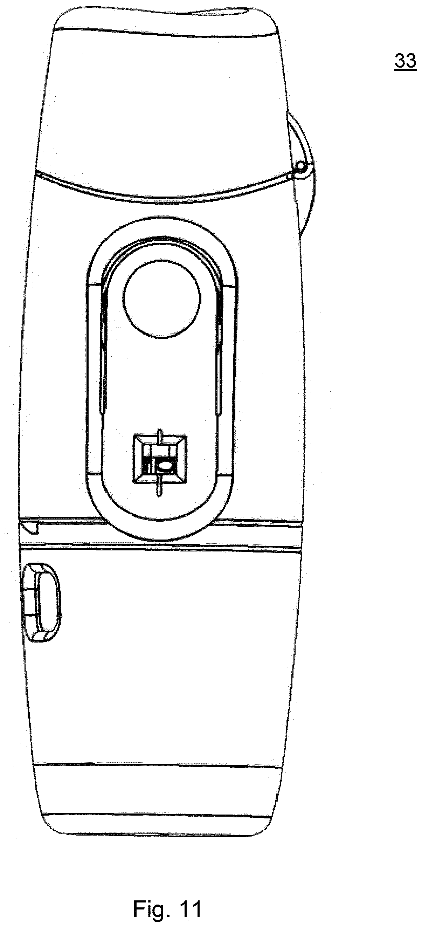

Fig. 11 shows an example of an aerosol dispenser 33, such as an inhaler. The aerosol dispenser 33 may be a medicament delivery device. The aerosol dispenser 33 comprises the spray nozzle device 29. When an aerosol is to be dispensed by the aerosol dispenser 33, the aerosol is created by the spray nozzle chip 1.

The aerosol dispenser 33 may comprise an electronics unit configured to power the pressure sensing device 12. The electronics unit may be configured to power the pressure sensing device 12 and to receive pressure measurement signals via the contact pads 12c. The electronics unit may be configured to power the reference pressure sensing device 19 and to receive pressure measurement signals via the reference contact pads 19c.

The electronics unit may be configured to process the pressure measurement signals from the pressure sensing device 12. For example, the electronics unit may be configured to determine the pressure applied to the spray membrane 7 in the spray orifice region based on the pressure measurement signals. The electronics unit may be configured to process the pressure measurement signals from the reference pressure sensing device 19. For example, the electronics unit may be configured to determine the suction force or pressure applied to the spray membrane 7 in the reference pressure region.

The electronics unit may according to one variation be configured to transmit the pressure measurement signals from the pressure sensing device 12 and/or the reference pressure sensing device 19 wirelessly to an external unit, such as a smart phone, a tablet computer or to a server in a cloud.

The present invention has mainly been described above with reference to a few examples. However, as is readily appreciated by a person skilled in the art, other embodiments than the ones disclosed above are equally possible within the scope of the present invention, as defined by the appended claims.

## Claims

1. A spray nozzle chip (1) comprising:
a substrate (3) having a spray side (3a) and a sieve side (3b),
a spray membrane (7) provided on the spray side (3a),
a sieve membrane (9) provided on the sieve side (3b),
wherein the spray membrane (7) is provided with spray orifices (11) and the sieve membrane (9) is provided with sieve orifices (13), wherein the substrate (3) has a fluid channel (15) which connects the spray orifices (11) with the sieve orifices (13), and
**characterized in that** the spray membrane (7) comprises a pressure sensing device (12) configured to measure deformation of the spray membrane (7) to obtain a measure of pressure on the spray membrane (7).

2. The spray nozzle chip (1) as claimed in claim 1, wherein the spray orifices (11) are provided in a spray orifice region of the spray membrane (7), and wherein the pressure sensing device (12) is configured to detect deformation of the spray orifice region.

3. The spray nozzle chip (1) as claimed in claim 1 or 2, wherein the pressure sensing device (12) comprises one of a strain gauge sensor, a capacitive pressure sensor and a piezoresistive pressure sensor.

4. The spray nozzle chip (1) as claimed in any of the preceding claims, wherein the pressure sensing device (12) is a microelectromechanical system, MEMS pressure sensor.

5. The spray nozzle chip (1) as claimed in any of the preceding claims, wherein the pressure sensing device (12) is arranged on the spray membrane (7).

6. The spray nozzle chip (1) as claimed in any of the preceding claims, comprising a reference pressure sensing device (19), wherein the substrate (3) has a closed cavity (17) which is delimited by a reference pressure region of the spray membrane (7), wherein the reference pressure sensing device (19) is configured to measure deformation of the reference pressure region.

7. The spray nozzle chip (1) as claimed in claim 6, wherein the cavity (17) is a vacuum cavity.

8. The spray nozzle chip (1) as claimed in claim 6 or 7, wherein the reference pressure sensing device (19) comprises one of a strain gauge sensor, a capacitive pressure sensor and a piezoresistive pressure sensor.

9. The spray nozzle chip (1) as claimed in any of claims 6-8, wherein the reference pressure sensing device (19) is a MEMS pressure sensor.

10. The spray nozzle chip (1) as claimed in any of claims 6-9, wherein the reference pressure sensing device (19) is arranged on the spray membrane (7).

11. A spray nozzle device (29) comprising:
the spray nozzle chip (1) as claimed in any of the preceding claims, and
a contact interface (21) configured to be electrically connected to the pressure sensing device (12) to supply power to and obtain pressure measurement signals from the pressure sensing device (12).

12. The spray nozzle device (29) as claimed in claim 11, comprising the spray nozzle chip (1) as claimed in any of claims 6-10, wherein the contact interface (21) is configured to be electrically connected to the reference pressure sensing device (19) to supply power to and obtain pressure measurement signals from the reference pressure sensing device (19).

13. An aerosol dispenser (33) comprising the spray nozzle device (29) as claimed in claim 11 or 12.

14. The aerosol dispenser (33) as claimed in claim 13, wherein the aerosol dispenser (33) is a medicament delivery device.

15. The aerosol dispenser (33) as claimed in claim 14, wherein the medicament delivery device is an inhaler or eye dispenser.

## Patentansprüche

1. Sprühdüsenchip (1), umfassend:
ein Substrat (3), das eine Sprühseite (3a) und eine Siebseite (3b) aufweist,
eine Sprühmembran (7), die auf der Sprühseite (3a) bereitgestellt ist,
eine Siebmembran (9), die auf der Siebseite (3b) bereitgestellt ist,
wobei die Sprühmembran (7) mit Sprühöffnungen (11) versehen ist und die Siebmembran (9) mit Sieböffnungen (13) versehen ist, wobei das Substrat (3) einen Fluidkanal (15) aufweist, der die Sprühöffnungen (11) mit den Sieböffnungen (13) verbindet, und
**dadurch gekennzeichnet, dass** die Sprühmembran (7) eine Druckerfassungsvorrichtung (12) umfasst, die konfiguriert ist, um eine Verformung der Sprühmembran (7) zu messen, um ein Maß an Druck auf der Sprühmembran (7) zu erhalten.

2. Sprühdüsenchip (1) nach Anspruch 1, wobei die Sprühöffnungen (11) in einem Sprühöffnungsbereich der Sprühmembran (7) bereitgestellt sind, und wobei die Druckerfassungsvorrichtung (12) konfiguriert ist, um die Verformung des Sprühöffnungsbereichs zu erfassen.

3. Sprühdüsenchip (1) nach Anspruch 1 oder 2, wobei die Druckerfassungsvorrichtung (12) eines von einem Dehnungsmesssensor, einem kapazitiven Drucksensor und einem piezoresistiven Drucksensor umfasst.

4. Sprühdüsenchip (1) nach einem der vorstehenden Ansprüche, wobei die Druckerfassungsvorrichtung (12) ein Drucksensor eines mikroelektromechanischen Systems, MEMS-Drucksensor, ist.

5. Sprühdüsenchip (1) nach einem der vorstehenden Ansprüche, wobei die Druckerfassungsvorrichtung (12) auf der Sprühmembran (7) angeordnet ist.

6. Sprühdüsenchip (1) nach einem der vorstehenden Ansprüche, umfassend eine Referenzdruckerfassungsvorrichtung (19), wobei das Substrat (3) einen geschlossenen Hohlraum (17) aufweist, der durch einen Referenzdruckbereich der Sprühmembran (7) begrenzt ist, wobei die Referenzdruckerfassungsvorrichtung (19) konfiguriert ist, um die Verformung des Referenzdruckbereichs zu messen.

7. Sprühdüsenchip (1) nach Anspruch 6, wobei der Hohlraum (17) ein Vakuumhohlraum ist.

8. Sprühdüsenchip (1) nach Anspruch 6 oder 7, wobei die Referenzdruckerfassungsvorrichtung (19) eines von einem Dehnungsmesssensor, einem kapazitiven Drucksensor und einem piezoresistiven Drucksensor umfasst.

9. Sprühdüsenchip (1) nach einem der Ansprüche 6 bis 8, wobei die Referenzdruckerfassungsvorrichtung (19) ein MEMS-Drucksensor ist.

10. Sprühdüsenchip (1) nach einem der Ansprüche 6 bis 9, wobei die Referenzdruckerfassungsvorrichtung (19) auf der Sprühmembran (7) angeordnet ist.

11. Sprühdüsenvorrichtung (29), umfassend:
den Sprühdüsenchip (1) nach einem der vorstehenden Ansprüche und
eine Kontaktschnittstelle (21), die konfiguriert ist, um mit der Druckerfassungsvorrichtung (12) elektrisch verbunden zu werden, um die Druckerfassungsvorrichtung (12) mit Strom zu versorgen und Druckmesssignale von ihr zu erhalten.

12. Sprühdüsenvorrichtung (29) nach Anspruch 11, umfassend den Sprühdüsenchip (1) nach einem der Ansprüche 6 bis 10, wobei die Kontaktschnittstelle (21) konfiguriert ist, um mit der Referenzdruckerfassungsvorrichtung (19) elektrisch verbunden zu werden, um die Referenzdruckerfassungsvorrichtung (19) mit Strom zu versorgen und Druckmesssignale von ihr zu erhalten.

13. Aerosolspender (33), umfassend die Sprühdüsenvorrichtung (29) nach Anspruch 11 oder 12.

14. Aerosolspender (33) nach Anspruch 13, wobei der Aerosolspender (33) eine Medikamentenabgabevorrichtung ist.

15. Aerosolspender (33) nach Anspruch 14, wobei die Medikamentenabgabevorrichtung ein Inhalator oder ein Spender für Augen ist.

## Revendications

1. Puce de buse de pulvérisation (1) comprenant :
un substrat (3) ayant un côté pulvérisation (3a) et un côté tamis (3b),
une membrane de pulvérisation (7) prévue sur le côté pulvérisation (3a),
une membrane de tamis (9) prévue sur le côté tamis (3b),
dans laquelle la membrane de pulvérisation (7) est pourvue d'orifices de pulvérisation (11) et la membrane de tamis (9) est pourvue d'orifices de tamis (13), dans laquelle le substrat (3) a un canal de fluide (15) qui relie les orifices de pulvérisation (11) aux orifices de tamis (13), et
**caractérisée en ce que** la membrane de pulvérisation (7) comprend un dispositif de détection de pression (12) conçu pour mesurer la déformation de la membrane de pulvérisation (7) afin d'obtenir une mesure de pression sur la membrane de pulvérisation (7).

2. Puce de buse de pulvérisation (1) selon la revendication 1, dans laquelle les orifices de pulvérisation (11) sont prévus dans une région d'orifice de pulvérisation de la membrane de pulvérisation (7), et dans laquelle le dispositif de détection de pression (12) est conçu pour détecter une déformation de la région d'orifice de pulvérisation.

3. Puce de buse de pulvérisation (1) selon la revendication 1 ou 2, dans laquelle le dispositif de détection de pression (12) comprend l'un parmi un capteur à jauge de contrainte, un capteur de pression capacitif et un capteur de pression piézorésistif.

4. Puce de buse de pulvérisation (1) selon l'une quelconque des revendications précédentes, dans laquelle le dispositif de détection de pression (12) est un capteur de pression de système microélectromécanique, MEMS.

5. Puce de buse de pulvérisation (1) selon l'une quelconque des revendications précédentes, dans laquelle le dispositif de détection de pression (12) est agencé sur la membrane de pulvérisation (7).

6. Puce de buse de pulvérisation (1) selon l'une quelconque des revendications précédentes, comprenant un dispositif de détection de pression de référence (19), dans laquelle le substrat (3) a une cavité fermée (17) qui est délimitée par une région de pression de référence de la membrane de pulvérisation (7), dans laquelle le dispositif de détection de pression de référence (19) est conçu pour mesurer une déformation de la région de pression de référence.

7. Puce de buse de pulvérisation (1) selon la revendication 6, dans laquelle la cavité (17) est une cavité à vide.

8. Puce de buse de pulvérisation (1) selon la revendication 6 ou 7, dans laquelle le dispositif de détection de pression de référence (19) comprend l'un parmi un capteur à jauge de contrainte, un capteur de pression capacitif et un capteur de pression piézorésistif.

9. Puce de buse de pulvérisation (1) selon l'une quelconque des revendications 6 à 8, dans laquelle le dispositif de détection de pression de référence (19) est un capteur de pression MEMS.

10. Puce de buse de pulvérisation (1) selon l'une quelconque des revendications 6 à 9, dans laquelle le dispositif de détection de pression de référence (19) est agencé sur la membrane de pulvérisation (7).

11. Dispositif de buse de pulvérisation (29) comprenant :
la puce de buse de pulvérisation (1) selon l'une quelconque des revendications précédentes, et
une interface de contact (21) conçue pour être connectée électriquement au dispositif de détection de pression (12) afin de fournir de l'énergie au dispositif de détection de pression (12) et d'obtenir des signaux de mesure de pression à partir de celui-ci.

12. Dispositif de buse de pulvérisation (29) selon la revendication 11, comprenant la puce de buse de pulvérisation (1) selon l'une quelconque des revendications 6 à 10, dans laquelle l'interface de contact (21) est conçue pour être connectée électriquement au dispositif de détection de pression de référence (19) afin de fournir de l'énergie au dispositif de détection de pression de référence (19) et d'obtenir des signaux de mesure de pression à partir de celui-ci.

13. Distributeur d'aérosol (33) comprenant le dispositif de buse de pulvérisation (29) selon la revendication 11 ou 12.

14. Distributeur d'aérosol (33) selon la revendication 13, dans lequel le distributeur d'aérosol (33) est un dispositif d'administration de médicament.

15. Distributeur d'aérosol (33) selon la revendication 14, dans lequel le dispositif d'administration de médicament est un inhalateur ou un distributeur oculaire.
